# EUROPEAN PATENT APPLICATION

(11) **EP 4 035 637 A1**
(43) Date of publication of application: **03.08.2022**
(21) Application number: 20868493.6
(22) Date of filing: 08.09.2020
(51) Int. Cl.: A61F 13/15, A61F 13/49, A61F 13/511

(54) **DISPOSABLE DIAPER**

(30) Priority: 26.09.2019 JP 2019174930
(71) Applicant: Daio Paper Corporation, Ehime 799-0492 (JP)
(72) Inventor: FURUKAWA, Masashi, Shikokuchuo-shi, Ehime 799-0431 (JP); OKADA, Yuki, Shikokuchuo-shi, Ehime 799-0431 (JP)
(74) Representative: Williams Powell
(86) International application number: PCT/JP2020/033903
(87) International publication number: WO 2021/059958

(57) **Abstract**

Provided is a disposable diaper which moisturizes the skin on the dorsal side part, crotch part, and ventral side part of a wearer and fit of which can be improved by causing a dorsal side-end flap thereof to be in close contact with the dorsal side part of the wearer.

The disposable diaper includes: a liquid pervious top sheet, a liquid impervious back sheet, an absorber disposed between the top sheet and the back sheet; and end flap parts formed on both sides of the absorber in a front-back direction, wherein a rectangular shaped stretchable sheet stretchable in a width direction is provided between the top sheet and the back sheet in a region that forms a dorsal side-end flap part, the stretchable sheet includes an inner sheet opposed to the top sheet, an outer sheet opposed to the back sheet, and elastic members stretching along the width direction and being disposed at predetermined intervals in the front-back direction between the inner sheet and the outer sheet, the elastic members are fixed via bonded portions formed at predetermined intervals in the width direction on an opposed body side surface of the inner sheet and on a body side surface of the outer sheet, lotion portions are formed at predetermined intervals in the width direction on a body side surface of the top sheet and extended in the front-back direction from a ventral side-end flap part toward the dorsal side-end flap part, and in a plan view, that section of the lotion portion which extends in the dorsal side-end flap part is provided such that the section overlaps with one side portion of one bonded portion and with the other side portion of the other bonded portion adjacent to the one bonded portion.

[Representative Drawing] Fig. 5

## Description

### Technical Field

The present invention relates to disposable diapers and particularly relates to a disposable diaper that moisturizes the skin of a wearer and improves fit of the disposable diaper to the wearer.

### Background Art

In order to reduce the adherence of excreted feces on a disposable diaper to the skin of a wearer, a technique has been known that lotion is applied to a body side surface of the top sheet on a dorsal side site (Patent Literature 1).

### Citation List

### Patent Literature

Patent Literature 1: JP 2010-75733 A

### Summary of Invention

### Technical Problem

However, according to the technique of Patent Literature 1, it is difficult to moisturize sufficiently the skin of the wearer on the crotch part and ventral side part, and in addition, there is a possibility that the excrement might be leaked from edges of the disposable diaper on the dorsal side.

Therefore, an object of the present invention is to provide a disposable diaper moisturizing the skin of a wearer on the dorsal side part, crotch part and ventral side part and permitting improved fit thereof to the wearer by causing a dorsal side-end flap of the disposable diaper to be in close contact with the dorsal side part of the wearer.

### Solution to Problem

The present invention, which provides solutions to the above problem, is described below.

A first aspect includes:
a liquid pervious top sheet, a liquid impervious back sheet, an absorber disposed between the top sheet and the back sheet; and
end flap parts formed on both sides of the absorber in a front-back direction, wherein
a rectangular shaped stretchable sheet stretchable in a width direction is provided between the top sheet and the back sheet in a region that forms a dorsal side-end flap part,
the stretchable sheet includes an inner sheet opposed to the top sheet, an outer sheet opposed to the back sheet, and elastic members stretching along the width direction and being disposed at predetermined intervals in the front-back direction between the inner sheet and the outer sheet,
the elastic members are fixed via bonded portions formed at predetermined intervals in the width direction on an opposed body side surface of the inner sheet and on a body side surface of the outer sheet,
lotion portions are formed at predetermined intervals in the width direction on a body side surface of the top sheet and extended in the front-back direction from a ventral side-end flap part toward the dorsal side-end flap part, and
in a plan view, that section of the lotion portion which extends in the dorsal side-end flap part is provided such that the section overlaps with one side portion of one bonded portion and with the other side portion of the other bonded portion adjacent to the one bonded portion.

A second aspect is the configuration of the first aspect wherein
a basis weight of lotion applied to the lotion portion is 2 to 20 g/mm².

A third aspect is the configuration of the first or second aspect wherein
a basis weight of lotion applied to that section of the lotion portion which extends in the dorsal side-end flap part is larger than a basis weight of lotion applied to that section of the lotion portion which extends on the absorber.

A fourth aspect is the configuration of any one of the first to third aspects wherein
in a plan view, a length in the width direction of that section of the lotion portion which extends in the dorsal side-end flap part is longer than a length in the width direction of that section of the lotion portion which extends on the absorber.

A fifth aspect is the configuration of any one of the first to fourth aspects wherein
the lotion is formed of water soluble glycerin.

### Advantageous Effects

According to the first aspect comprising:
a liquid pervious top sheet, a liquid impervious back sheet, an absorber disposed between the top sheet and the back sheet; and
end flap parts formed outwardly on both sides of the absorber in a front-back direction, since
a rectangular shaped stretchable sheet stretchable in a width direction is provided between the top sheet and the back sheet in a region that forms a dorsal side-end flap part,
the stretchable sheet includes an inner sheet opposed to the top sheet, an outer sheet opposed to the back sheet, and elastic members stretching along the width direction and being disposed at predetermined intervals in the front-back direction between the inner sheet and the outer sheet,
the elastic members are fixed via bonded portions formed at predetermined intervals in the width direction on an opposed body side surface of the inner sheet and on a body side surface of the outer sheet,
lotion portions are formed at predetermined intervals in the width direction on a body side surface of the top sheet and extended in the front-back direction from a ventral side-end flap part toward the dorsal side-end flap part, and
in a plan view, that section of the lotion portion which extends in the dorsal side-end flap part is provided such that the section overlaps with one side portion of one bonded portion and with the other side portion of the other bonded portion adjacent to the one bonded portion,
it is possible to moisturize the skin of a wearer and increase an extended height of a pleat formed on the dorsal side-end flap EF, resulting in improved fit of the disposable diaper to the waist part on the dorsal side of the wearer.

According to the second aspect, since
a basis weight of lotion applied to the lotion portion is 2 to 20 g/mm²,
it is possible to moisturize the skin of the wearer efficiently in addition to the effects by the first aspect.

According to the third aspect, since
a basis weight of lotion applied to that section of the lotion portion which extends in the dorsal side-end flap part is larger than a basis weight of lotion applied to that section of the lotion portion which extends on the absorber,
it is possible to decrease surely adhesive forces at the other side portion of one bonded portion and at one side portion of the other bonded portion so that the extended height of the pleat formed on the dorsal side-end flap EF is more increased, resulting in more improved fit of the disposable diaper to the waist part on the dorsal side of the wearer, in addition to the effects by the first or second aspect.

According to the fourth aspect, since
in a plan view, a length in the width direction of that section of the lotion portion which extends in the dorsal side-end flap part is longer than a length in the width direction of that section of the lotion portion which extends on the absorber,
it is possible to increase an area of the other side portion of one bonded portion and an area of one side portion of the other bonded portion that overlap with the lotion portion so that the extended height of the pleat formed on the dorsal side-end flap EF is more increased, resulting in more improved fit of the disposable diaper to the waist part on the dorsal side of the wearer, in addition to the effects by any one of the first to third aspects.

According to the fifth aspect, since
the lotion is formed of water soluble glycerin,
it is possible to capture water from the surrounding atmosphere and moisturize the skin of the wearer more fully, in addition to the effects by any one of the first to fourth aspects.

### Brief Description of Drawings

Fig. 1 is a plan view illustrating a disposable diaper on a body side.
Fig. 2 is a plan view illustrating a disposable diaper on an opposed body side.
Fig. 3 is a cross-sectional view taken along line X1-X1 in Fig. 1.
Fig. 4 is a plan view of a stretchable sheet.
Fig. 5 is a diagram illustrating a mode in which lotion is applied.
Fig. 6 is a diagram illustrating a mode in which lotion is applied in the first embodiment.
Fig. 7 is a cross-sectional view taken along line X2-X2 in Fig. 6 and a diagram illustrating a basis weight of applied lotion.
Fig. 8 is a diagram illustrating a mode in which lotion is applied in the second embodiment.
Fig. 9 is a cross-sectional view taken along line X2-X2 in Fig. 8 and a diagram illustrating a basis weight of applied lotion.
Fig. 10 is a diagram illustrating a mode in which lotion is applied in the third embodiment.
Fig. 11 is a cross-sectional view taken along line X2-X2 in Fig. 10 and a diagram illustrating a basis weight of applied lotion.
Fig. 12 is a diagram illustrating a mode in which lotion is applied in the fourth embodiment.
Fig. 13 is a cross-sectional view taken along line X2-X2 in Fig. 12 and a diagram illustrating a basis weight of applied lotion.
Fig. 14 is a diagram illustrating a stretchable sheet before lotion is applied.
Fig. 15 is a diagram illustrating a stretchable sheet after lotion is applied.

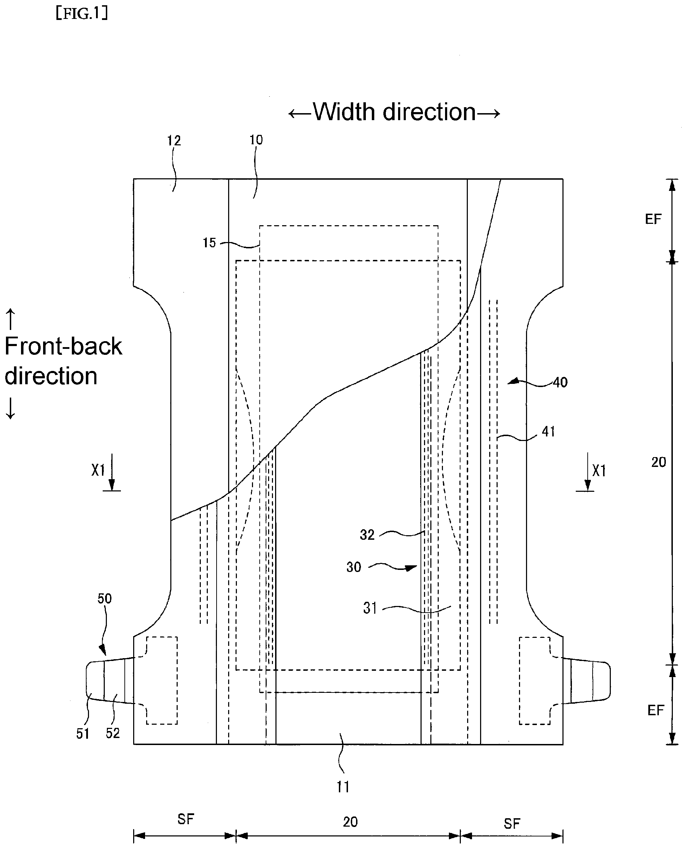

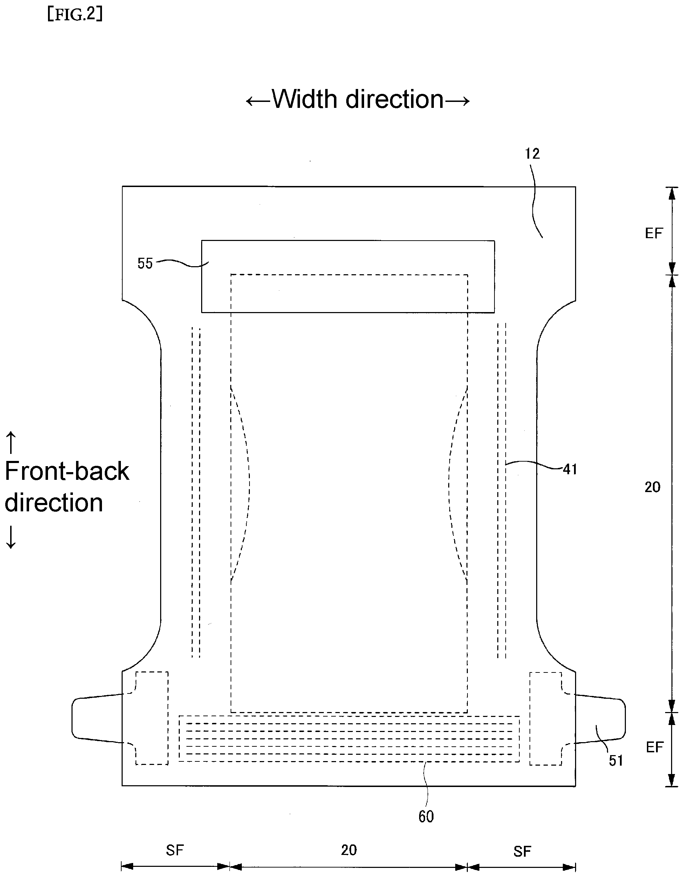

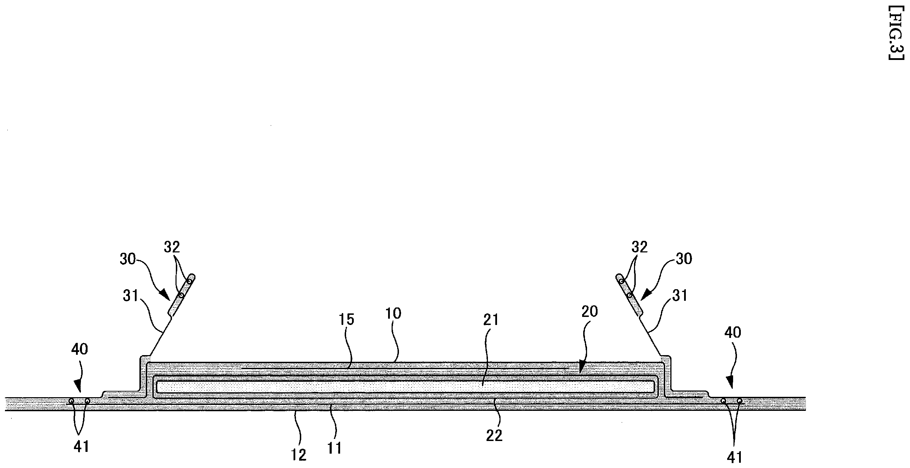

As illustrated in Figs. 1 to 3, a disposable diaper includes a liquid pervious top sheet 10 provided on a body side thereof, a liquid impervious back sheet 11 provided on an opposed body side thereof, and an absorbent element 20 provided between the top sheet 10 and the back sheet 11. The absorbent element 20 includes an absorber 21 and a wrapping sheet 22 wrapping the absorber 21.

An outer sheet (external sheet) 12 is provided on the opposed body side of the back sheet 11. It is preferable that an intermediate sheet 15 is provided between the top sheet 10 and the absorbent element 20 for transporting excrement having passed through the top sheet 10 into the absorbent element 20 and for preventing of returning of the excrement.

Three-dimensional gathers 30 are provided outwardly on both sides of the absorbent element 20 in a width direction such that the three-dimensional gathers 30 are spaced from each other with a predetermined distance therebetween for preventing of leaking of the excrement to the outside . Each three-dimensional gather 30 includes a gather sheet 31 being substantially continuous in the width direction and an elongated elastically stretchable member 32 being fixed in a stretched state along a front-back direction of the gather sheet 31.

Flat gathers 40 are formed outside from respective base portions of the both three-dimensional gathers 30 in the width direction such that the flat gathers 40 are spaced from each other with a predetermined distance therebetween for preventing of leaking of excreted urine to the outside. Each flat gather 40 includes the back sheet 11, the gather sheet 31, the outer sheet 12, and an elongated elastically stretchable member 41 fixed in a stretched state along the front-back direction between the back sheet 11 and the gather sheet 31.

End flap parts EF are formed on both sides of the absorbent element 20 in the front-back direction, respectively, while side flap parts SF are formed on both sides of the absorbent element 20 in the width direction.

A stretchable sheet 60 described later is provided between the top sheet 10 and the outer sheet 12 in a region that defines a dorsal side-end flap part EF.

Fastening tapes 50 are provided in both dorsal side-side flap parts SF in the width direction respectively. Each fastening tape 50 includes a base material 51 fixed to the side flap SF and an engagement portion 52 provided on the body side of the base material 51.

A rectangular shaped target sheet 55 with a predetermined width in the front-back direction is provided on the opposed body side of the external sheet 12 on the ventral side so as to extend in the width direction for engaging the engagement portions 52 of the fastening tapes 50 to the target sheet 55.

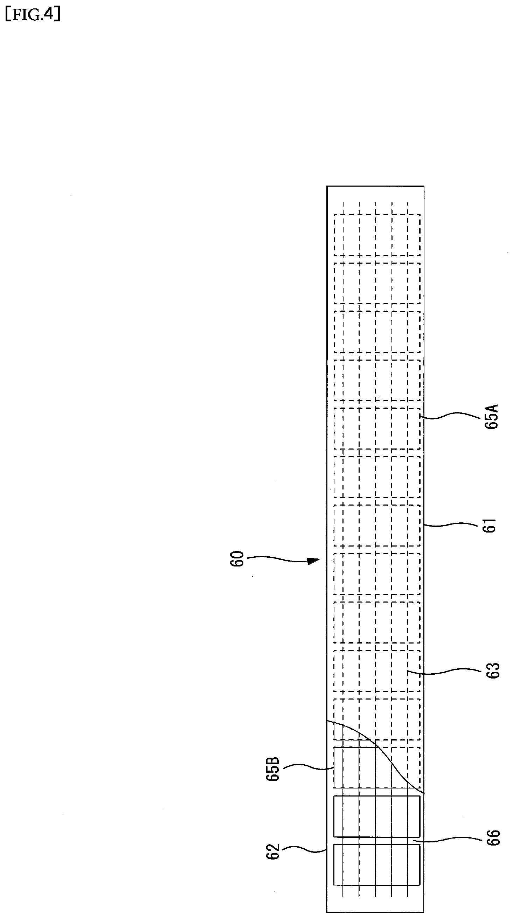

As illustrated in Fig. 4, the stretchable sheet 60 includes an inner sheet 61 opposed to the top sheet 10, an outer sheet 62 opposed to the outer sheet 12, and elongated elastically stretchable members (referred as"elastic members" in claim) 63 fixed in a stretched state along the width direction between the inner sheet 61 and the outer sheet 62.

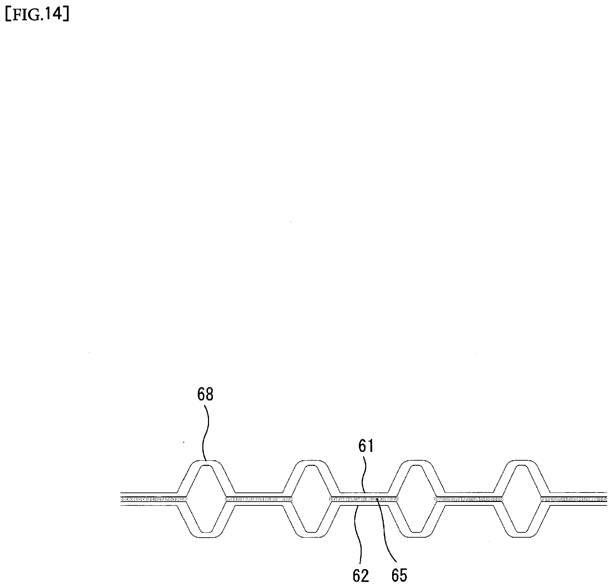

The elastically stretchable members 63 are fixed on rectangular shaped first bonded portions 65A formed at predetermined intervals in the width direction on an opposed body side surface of the inner sheet 61 and on second bonded portions 65B formed at predetermined intervals in the width direction on a body side surface of the outer sheet 62. By doing so, as illustrated in Fig. 14, substantially mountain shaped pleats 68 are formed on the body side surface and on the opposed body side surface of the stretchable sheet 60, resulting in that the disposable diaper can be fit to the waist part on the dorsal side of a wearer. The first bonded portions 65A and the second bonded portions 65B may be formed by applying a hot melt adhesive. In the present specification, the first bonded portions 65A and the second bonded portions 65B are referred totally as bonded portions 65 while spaces sandwiched between two adjacent bonded portions 65 are referred as non-bonded portions 66.

Alternatively, the bonded portions 65 are not formed on the inner sheet 61 or the outer sheet 62, instead, it is possible to form the bonded portions 65 on a periphery of the elastically stretchable member 63 at predetermined intervals in the width direction for fixing the inner sheet 61 and the outer sheet 62 to the elastically stretchable member 63.

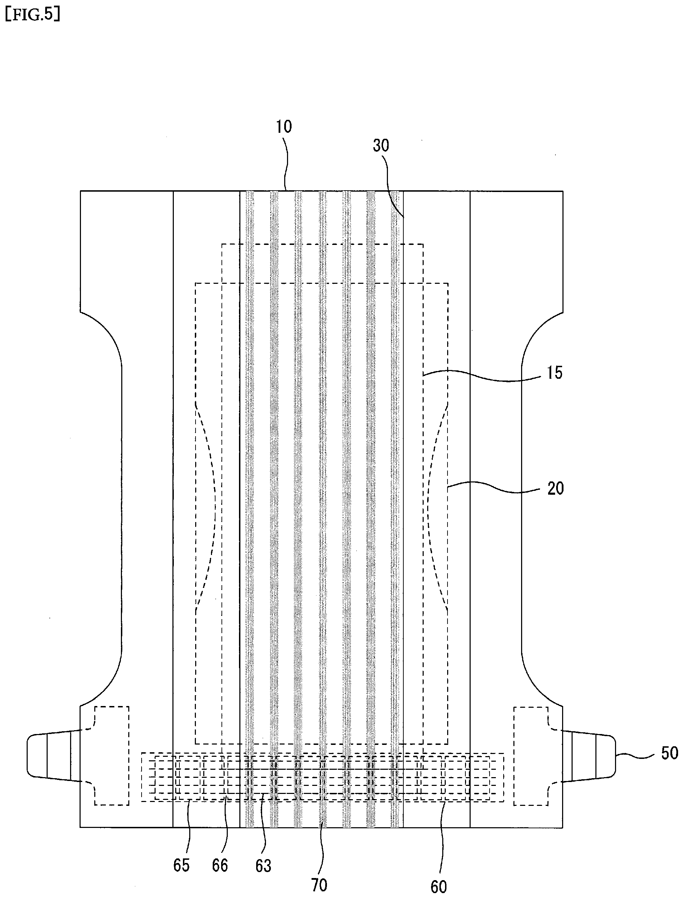

As illustrated in Fig. 5, in a space between the three-dimensional gathers 30 on the body side surface of the top sheet 10, lotion portions 70 are formed so as to extend in the front-back direction at predetermined intervals in the width direction. The lotion portions 70 are preferably formed so as to extend from a ventral side-end flap part EF toward a dorsal side-end flap part EF. By doing so, smooth contact may be performed at contact portions between the disposable diaper and the wearer such as between the ventral side-end flap part EF and the waist part on the ventral part of the wearer, and between the dorsal side-end flap part EF and the waist part on the dorsal side of the wearer, resulting in decreased damage to the skin of the wearer. The lotion portions 70 may be formed by application or transfer of lotion.

An area rate of the lotion portions 70, i.e., a rate of an area of the lotion portions 70 with respect to an area of the space between the both three-dimensional gathers 30 on the body side surface of the top sheet 10 is preferably 20% to 70%. When the rate is less than 20%, it is difficult to form the lotion portions 70 continuing in the front-back direction at the predetermined intervals in the width direction, while when the rate exceeds 70%, there is a possibility of too much lotion for moisturizing the skin of the wearer.

As the lotion, water soluble glycerin may be used, particularly, it is preferable to prepare the lotion by diluting glycerin by a predetermined amount of aqueous solution. Such lotion may moisturize the skin of the wearer by capturing water from the surrounding atmosphere. Further, it is possible to make the lotion moisture-rich by containing hyaluronic acid, collagen and ceramide in addition to the glycerin in the lotion. In this way, the lotion can be designed appropriately in accordance with when and where the disposable diaper is used. Additionally, it is also possible to provide barrier functions to the skin of the wearer by adding oily vaseline or the like to the lotion.

### <First embodiment>

The lotion portion 70 in the first embodiment will now be described.

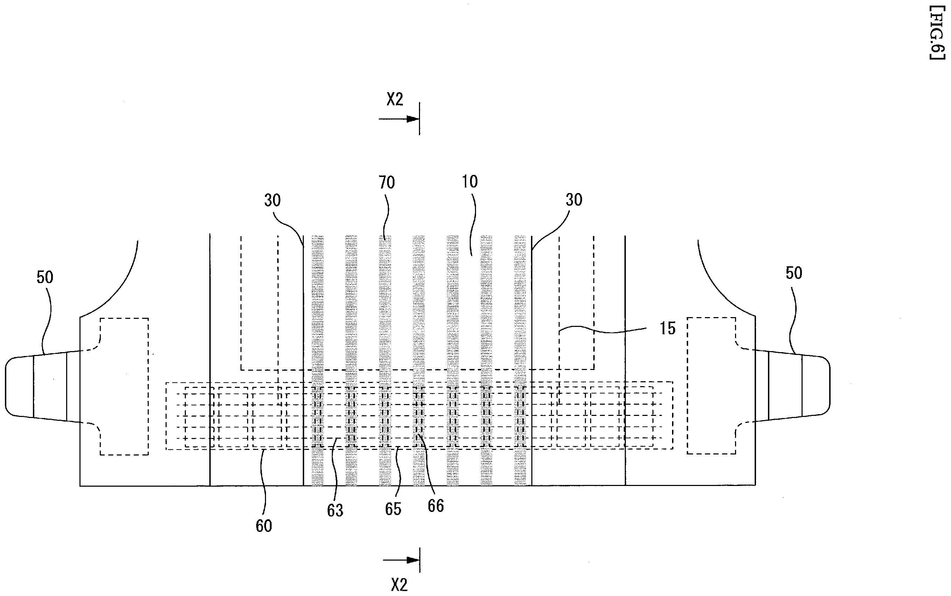

As illustrated in Fig. 6, the lotion portions 70 are formed so as to extend in the front-back direction on sites corresponding to the non-bonded portions 66 extending in the front-back direction on the stretchable sheet 60 disposed in the dorsal side-end flap part EF. The lotion portion 70 is formed such that the left side portion of the lotion portion 70 in the width direction overlaps with the right side portion of the bonded portion 65 that is formed outwardly on the left side of the non-bonded portion 66, while the right side portion of the lotion portion 70 in the width direction overlaps with the left side portion of the bonded portion 65 that is formed outwardly on the right side of the above non-bonded portion 66.

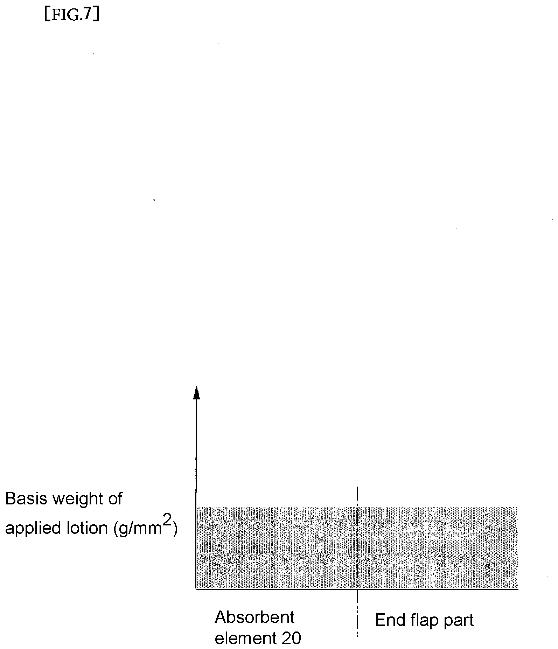

As illustrated in Fig. 7, the amount of the lotion applied to the lotion portion 70 is continued to be constant, for example 2 g/mm², from the ventral side-end flap part EF to the dorsal side-end flap part EF on the top sheet 10.

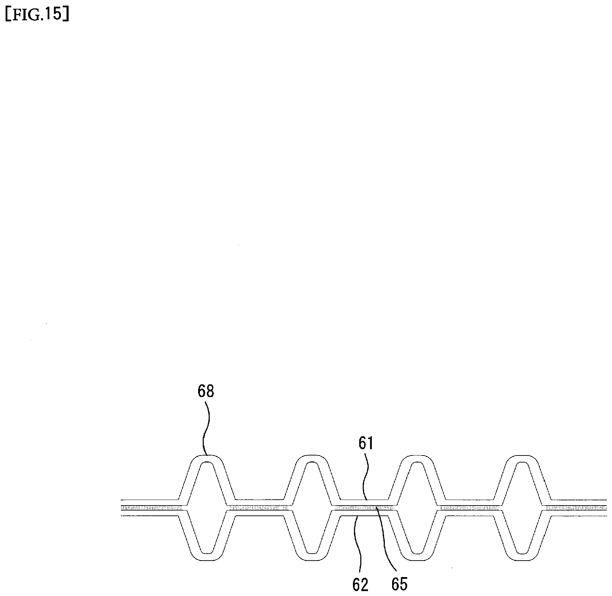

By doing so, with respect to adjacent two bonded portions 65, since adhesion forces are decreased at the right side portion of one bonded portion 65 that overlaps with the left side portion of the lotion portion 70 in the width direction and at the left side portion of the other bonded portion 65 that overlaps with the right side portion of the above lotion portion 70 in the width direction, it becomes possible for the pleat 68 formed on the body side surface of the stretchable sheet 60 to extend more toward the body of the wearer as illustrated in Fig. 15, resulting in more improved fit of the disposable diaper to the waist part on the dorsal side of the wearer.

The amount of lotion applied to the lotion portion 70 is preferably 2 to 20 g/mm². When the amount is less than 2 g/mm², it is difficult to moisturize the skin of the wearer while when the amount exceeds 20 g/mm², there is a possibility of too much lotion for moisturizing the skin of the wearer.

### <Second embodiment>

Next, the lotion portion 70 in the second embodiment will be described. The same parts as those related to the lotion portion 70 according to the first embodiment are denoted by the same reference signs, and description thereof will be omitted.

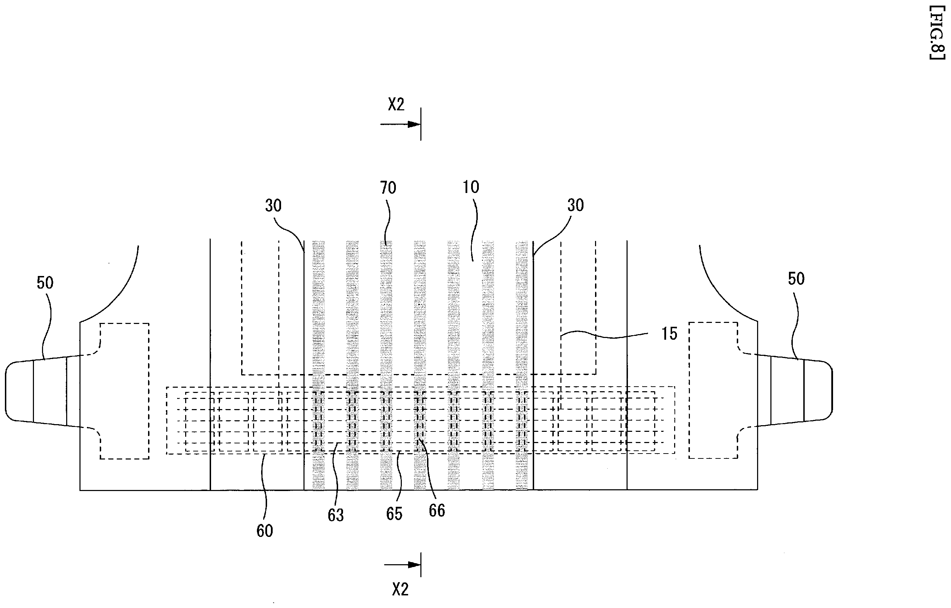

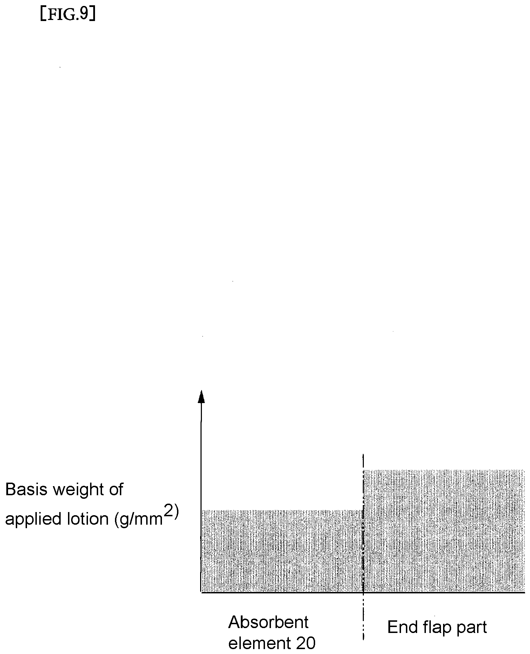

As illustrated in Fig. 9, the amount of the lotion applied to the lotion portion 70 is, for example 2 g/mm², from the ventral side-end flap part EF to the lower edge on the dorsal side of the absorbent element 20 on the top sheet 10, and for example 3 g/mm², in the dorsal side-end flap part EF on the top sheet 10. Since Fig. 8 is the same as Fig. 6, description thereof is omitted.

By doing so, an adhesion force in the right side portion of one bonded portion 65 that overlaps with the left side portion of the lotion portion 70 in the width direction and an adhesion force in the left side portion of the other bonded portion 65 that overlaps with the right side portion of the above lotion portion 70 in the width direction are decreased to extend uniformly the pleat 68 formed on the body side surface of the stretchable sheet 60 to the body of the wearer, resulting in reliable fit of the disposable diaper to the waist part on the dorsal side of the wearer.

### <Third embodiment>

Next, the lotion portion 70 in the third embodiment will be described. The same parts as those related to the lotion portion 70 according to the first embodiment are denoted by the same reference signs, and description thereof will be omitted.

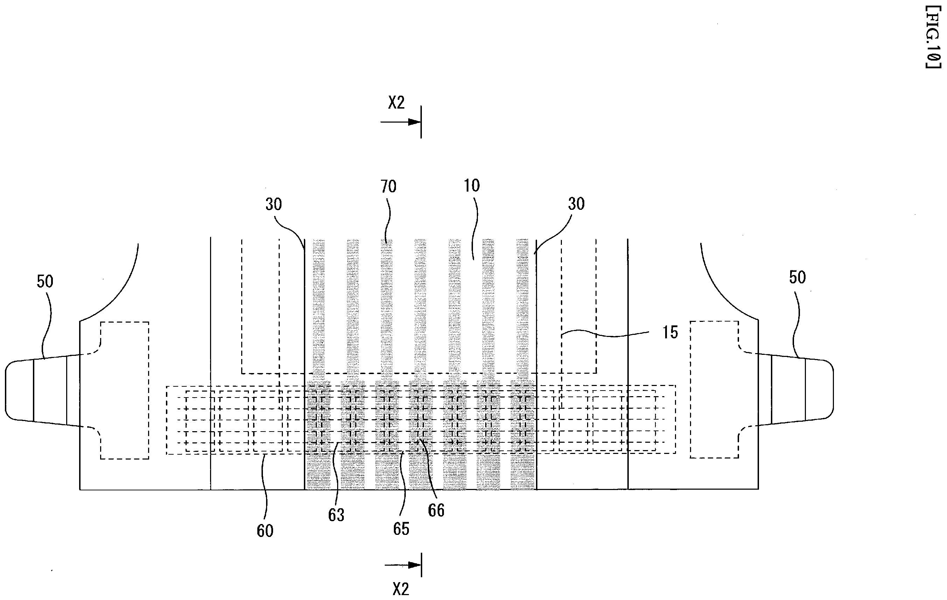

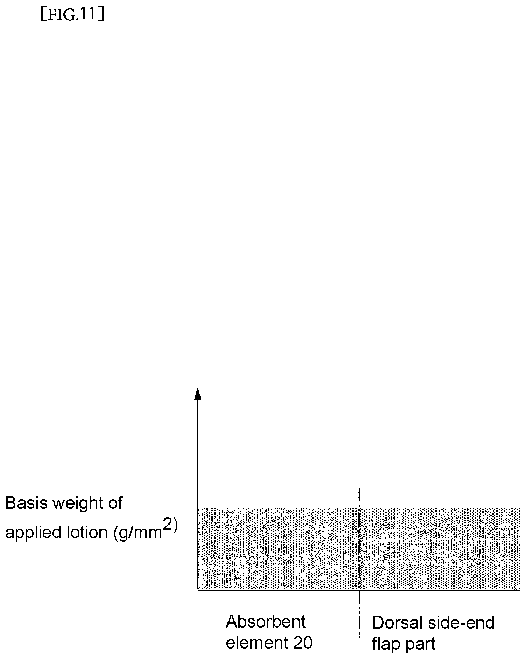

As illustrated in Fig. 10, the length in the width direction of the lotion portion 70 formed on the body side surface of the dorsal side-end flap part EF on the top sheet 10 is made to be longer than the length in the width direction of the lotion portion 70 formed on the body side surface of the ventral side-end flap part EF on the top sheet 10. Since Fig. 11 is the same as Fig. 7, description thereof is omitted.

By doing so, the area of the right side portion of one bonded portion 65 that overlaps with the left side portion of the lotion portion 70 in the width direction and the area of the left side portion of the other bonded portion 65 that overlaps with the right side portion of the above lotion portion 70 in the width direction can be enlarged to extend the pleat 68 formed on the body side surface of the stretchable sheet 60 further to the body side of the wearer, resulting in further fit of the disposable diaper to the waist part on the dorsal side of the wearer.

In order to fix the elastically stretchable members 63 to the inner sheet 61 and to the outer sheet 62 through the bonded portions 65, the rate of area of portions of the bonded portions 65 where the bonded portions 65 and the lotion portions 70 are not overlapped each other with respect to the area of the bonded portions 65 is preferably 30% to 40% or more.

### <Fourth embodiment>

Next, the lotion portion 70 in the fourth embodiment will be described. The same parts as those related to the lotion portion 70 according to the third embodiment are denoted by the same reference signs, and description thereof will be omitted.

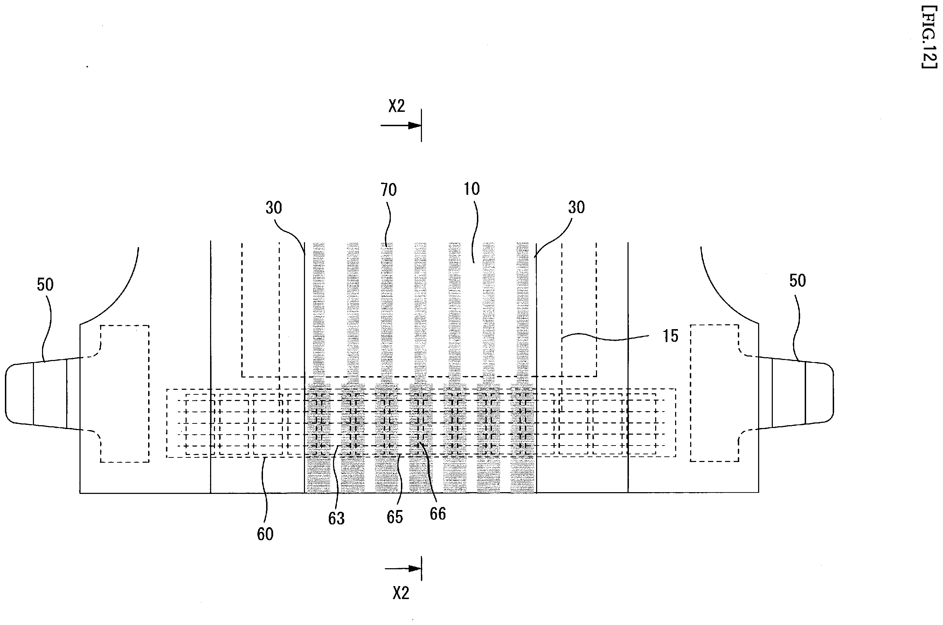

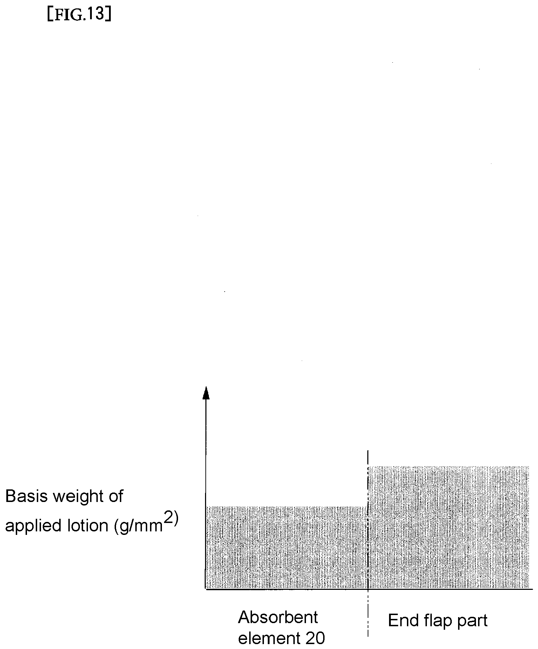

As illustrated in Fig. 13, the amount of the lotion applied to the lotion portion 70 is, for example 2 g/mm², from the ventral side-end flap part EF to the lower edge on the dorsal side of the absorbent element 20 on the top sheet 10, and for example 3 g/mm², in the dorsal side-end flap part EF on the top sheet 10. Since Fig. 12 is the same as Fig. 10, description thereof is omitted.

By doing so, an adhesion force in the right side portion of one bonded portion 65 that overlaps with the left side portion of the lotion portion 70 in the width direction and an adhesion force in the left side portion of the other bonded portion 65 that overlaps with the right side portion of the above lotion portion 70 in the width direction are decreased to extend uniformly the pleat 68 formed on the body side surface of the stretchable sheet 60 to the body of the wearer, resulting in reliable fit of the disposable diaper to the waist part on the dorsal side of the wearer.

Now, materials and features of components such as the top sheet 10 will be described in order below.

### (Top Sheet)

The top sheet 10 is formed of a perforated or unperforated nonwoven fabric, porous plastic sheet or the like. Among of them, the type of constituent fibers of the nonwoven fabric is not particularly limited. Examples of the constituent fibers can include synthetic fibers such as polyolefin type such as polyethylene and polypropylene, polyester type, and polyamide type, regenerated fibers such as rayon and cupra, natural fibers such as cotton, mixed fibers and composite fibers in which two or more of these are used. Further, the nonwoven fabric may be manufactured by any processing. Examples of the processing method include known methods such as a spun lace method, a spun bond method, a thermal bond method, a meltblown method, a needle punch method, an air-through method, and a point bond method. For example, the spun lace method is preferable when flexibility and drapability are required, and the thermal bonding method is preferable when bulkiness and softness are required.

### (Back Sheet)

The material of the back sheet 11 is a polyolefin-based resin such as polyethylene or polypropylene, a laminated nonwoven fabric obtained by stacking a nonwoven fabric on a polyethylene sheet or the like, a nonwoven fabric in which liquid impermeability is substantially secured through a water proof film (in this case, the back sheet is formed by the water proof film and the nonwoven fabric) can be exemplified. Obviously, besides these materials, in recent years, liquid-impervious and moisture-pervious materials which have been favorably used from the standpoint of prevention of stuffiness can also be exemplified. As a sheet of this liquid-impervious and moisture-pervious material, for example, a microporous sheet can be exemplified which is obtained by kneading an inorganic filler in a polyolefin-based resin such as polyethylene or polypropylene, molding the kneaded mixture into a sheet, and stretching the sheet in one or two axial directions. Further, nonwoven fabrics using micro denier fibers and a sheet that is liquid-impervious without using a water proof film can also be used as the back sheet 11. The sheet has a high leak proof obtained by heating or applying pressure to reduce air gaps of fibers, and a liquid impermeability obtained by applying a super absorbent polymer, a hydrophobic resin, or a water repellent agent.

### (External Sheet)

The outer sheet 12 is a component of the disposable diaper for supporting the absorbent element 20 and for attaching the disposable diaper to the wearer. The outer sheet 12 has an hourglass shape in which the central portion in the front-back direction is narrowed on the both sides thereof so as to surround the legs of the wearer.

A nonwoven fabric is suitable for the outer sheet 12. The type of the nonwoven fabric is not particularly limited. As a raw material fiber, for example, in addition to synthetic fibers such as olefin type such as polyethylene or polypropylene, polyester type, and polyamide type, regenerated fibers such as rayon and cupra, and natural fibers such as cotton can be used. As a processing method, a spun lace method, a spun bond method, a thermal bond method, an air through method, a needle punch method, and the like can be used. However, a long-fiber nonwoven fabric such as a spunbonded nonwoven fabric, an SMS nonwoven fabric, and an SMMS nonwoven fabric are preferable in that both good texture and strength can be compatible. In addition to using a single piece of nonwoven fabric, it is also possible to use stacked multiple nonwoven fabrics. In the latter case, it is preferable that the nonwoven fabrics are adhered to each other with adhesive or the like. When a nonwoven fabric is used, the basis weight of the fiber is desirably 10 to 50 g/m², particularly desirably 15 to 30 g/m².

### (Intermediate Sheet)

A material of the intermediate sheet 15 is a similar material to that of the top sheet 10. The intermediate sheet 15 is preferably bonded to the top sheet 10. In a case where heat embossing or ultrasonic welding is used for the bonding, a material of the intermediate sheet 15 preferably has approximately the same melting point as the top sheet 10. When the nonwoven fabric is used for the intermediate sheet 15, it is preferable that the fineness of fibers of the nonwoven fabric is about 2.0 to 5.0 dtex.

### (Absorber)

The absorber 21 can be formed by an assembly of fibers. As this fiber assembly, in addition to those obtained by accumulating short fibers such as fluff pulps or synthetic fibers, a filament assembly obtained by opening a tow (fiber bundle) of synthetic fibers such as cellulose acetate as necessary can also be used. In a case where fluff pulps or short fibers are accumulated, a fiber basis weight may be, for example, about 100 to 300 g/m². In a case of a filament assembly, a fiber basis weight may be, for example, about 30 to 120 g/m². In a case of a synthetic fiber, a fineness is, for example, 1 to 16 dtex, preferably 1 to 10 dtex, and more preferably 1 to 5 dtex. In a case of a filament assembly, the filament may be formed of non-crimped fibers but is preferably formed of crimped fibers. The degree of crimp of the crimped fibers may be, for example, about 5 to 75, preferably 10 to 50, and more preferably 15 to 50 per 1 inch. A uniformly crimped fiber is often used.

Preferably, the absorber 21 contains super absorbent polymer particles. More preferably, the super absorbent polymer particles (SAP particles) are dispersed across the substantially entire thickness of the fiber assembly in at least the liquid receiving region.

If the SAP particles are absent or present in slight amounts in the upper, lower, or intermediate portions of the absorber 21, the SAP particles should not be referred to as being "dispersed across the entire thickness". The form in which the SAP particles are "dispersed across the entire thickness," includes the form in which the SAP particles are "uniformly" dispersed across the entire thickness of the fiber assembly, and the form in which the SAP particles are "unevenly" dispersed in the upper, lower, and intermediate portions but still dispersed in the upper, lower, and intermediate portions. The form in which the SAP particles are "dispersed across the entire thickness" does not exclude the form in which some of the SAP particles remain on the surface of the fiber assembly without intrusion into the fiber assembly or the form in which some of the SAP particles reside on the wrapping sheet 22 after passing through the fiber assembly.

Super absorbent polymer particles include "powder" in addition to "particles." The super absorbent polymer particles may be the same as that of particles for general use in this type of absorbent article and preferably have a diameter of 1000 µm or less, more preferably 150 to 400 µm. There is no particular limitation on the material for the super absorbent polymer particles. Preferably, the material has water absorption capacity of 40 g/g or more. Examples of the super absorbent polymer particles are based on starch, cellulose, and synthetic polymer, such as graft copolymer of starch and acrylic acid (salt), saponified copolymers of starch and polyacrylonitrile, cross-linked sodium carboxymethyl cellulose, and acrylic acid (salt) copolymer. Preferably, the superabsorbent polymer particles are in the form of generally used particulate. Alternatively, the super absorbent polymer particles may have another form.

Preferably, the used super absorbent polymer particles have an absorption rate of 70 seconds or less, particularly 40 seconds or less. When the absorption rate is too slow, so called returning is easily caused, precisely the liquid, which has been supplied into the absorber 21, is more likely to flow back from the absorber 21 to the outside of the absorber 21.

The basis weight of the super absorbent polymer particles can be appropriately determined in accordance with the required absorption volume of the absorber 21 depending on use. Thus, although it is not necessarily appropriate to suggest, the basis weight may be within the range of 50 to 350 g/m². A basis weight of polymers of less than 50 g/m² fails to achieve a sufficient absorption volume. A basis weight of polymers of more than 350 g/m² saturates the absorption volume and provides the wearer a feeling of strangeness due to the granular texture generated by excess super absorbent polymer particles.

### (Wrapping Sheet)

Examples of the material for the wrapping sheet 22 include tissue paper, in particular, crepe paper, non-woven fabric, polyethylene laminated non-woven fabric, and a perforated sheet. Preferably, the sheet is configured such that the superabsorbent polymer particles do not pass through the sheet. In the case where non-woven fabric is used in place of crepe paper, hydrophilic SMMS (spunbond/melt blown/melt blown/spunbond) non-woven fabric is preferred. Examples of the material thereof include polypropylene and polyethylene/polypropylene. The fiber basis weight is preferably within the range of 5 to 40 g/m², more preferably 10 to 30 g/m².

### (Three-dimensional Gather)

As the gather sheet 31 of the three-dimensional gather 30, a water repellent nonwoven fabric can be used, and rubber thread and the like can be used as the elastically stretchable member 32. One or a plurality of the elastically stretchable members can be provided for each side.

The opposed body side surface of the gather sheet 31 has a fixed start point in the width direction on the side portion of the top sheet 10. A portion outside in the width direction from this fixed start point is fixed with hot melt adhesive or the like to the side portion of the back sheet 11 and to the side portion of the outer sheet 12 positioned outside the side portion of the back sheet 11.

In the periphery of the leg, the inside in the width direction from the fixed start point of each three-dimensional gather 30 is fixed on the top sheet 30 at both ends of the product in the front-back direction. However, the portion therebetween is a non-fixed free portion erected by contraction force of the elastically stretchable member 32. Since the diaper is attached to the body of the wearer in a boat shape at the time of wearing the diaper, and the contraction force of the elastically stretchable member 32 acts, the three-dimensional gather 30 erects by the contraction force of the elastically stretchable member 32 and come in close contact with the leg. As a result, so-called lateral leakage from around the leg is prevented.

### (Flat Gather)

The elastically stretchable member 41 composed of a rubber thread and the like is fixed in a stretched state along the front-back direction between the gather sheet 31 and the back sheet 11. One or a plurality of the elastically stretchable members 41 can be provided for each side.

### (Fastening Tape)

In the fastening tape 50, the basis portion of a base material 51 is fixed between the gather sheet 31 and the outer sheet 12 with hot melt adhesive or the like. The sheet base material 51 is formed of a nonwoven fabric, a plastic film, a polyethylene laminated nonwoven fabric, paper, or a composite material of these materials.

The engagement portion 52 is composed of a hook member of a mechanical fastener. The hook member has a large number of engagement projections on its outer surface side. The engagement projection has (A) a check mark shape, (B) a J shape, (C) a mushroom shape, (D) a T shape, and (E) a double J shape (a shape bonded back to back of a J shape), but may have any shape. Obviously, an adhesive material layer can also be provided as an engagement portion of the fastening tape 50.

### (Target Sheet)

The target sheet 55 is formed of a plastic film, a nonwoven fabric or the like, having many loop yarns provided on the surface of the target sheet.

### (Stretchable Sheet on Dorsal Side)

The stretchable sheet 60 is a sheet which stretches the dorsal side-end frap portion EF so as to cause the dorsal side-end flap part EF to be in close contact around the dorsal side part of the wearer. As illustrated in Fig. 4, the stretchable sheet 60 on the dorsal side includes the inner sheet 61 composed of a nonwoven fabric, the outer sheet 62 composed of a nonwoven fabric and the plurality of elongated elastically stretchable members 63 extending in the width direction and being disposed at predetermined intervals in the front-back direction between the inner sheet 61 and the outer sheet 62. As the elastically stretchable members 63, rubber threads having the fineness of 470 to 620 dtex are provided so as to extend at the stretch rate of 200% to 250%.

Both side portions of the stretchable sheet 60 in the width direction are located in the vicinity of both side portions of a pair of right and left gather sheets, while both side portions of the elastically stretchable member 63 in the width direction are located on both side portions of the back sheet 11.

### <Description of Terms Used in Specification>

The following terms used in the specification should be understood to have the meanings defined below unless otherwise specified in the specification.

"Front-back (Longitudinal) direction" refers to the direction connecting the ventral side (front side) and the dorsal side (back side), and "width direction" refers to the direction orthogonal to the front-back direction (right-left direction).

"Front surface side" refers to a side closer to the skin of the wearer, while "back surface side" refers to a side farther from the skin of the wearer. "Front side surface" refers to a surface of a member closer to the skin of the wearer, while "back side surface" refers to a surface of the member farther from the skin of the wearer.

"Machine direction: MD" and "cross direction: CD" mean the flow direction (MD) in a manufacturing equipment and the lateral direction (CD) orthogonal to the flow direction, and either one is the front-back direction, and the other is the width direction of a product. The MD of a nonwoven fabric is the direction of fiber orientation of the nonwoven fabric. "Fiber orientation" is a direction along which a fiber of a nonwoven fabric runs and determined by, for example, a measurement method in accordance with the fiber orientation test method based on the zero distance tensile strength of TAPPI standard method T471 and a simple measurement method for determining the fiber orientation direction from the tensile strength ratio of the front-back direction to the width direction.

"Spread" refers to a flat spread state without contraction or looseness.

"Stretch rate" refers to a value when the natural length is set to 100%. For example, the stretch rate of 200% has the same meaning as the elongation ratio of 2.

"Gel strength" is measured as follows: 1.0 g of super absorbent polymers are added to 49.0 g of artificial urine and the mixture is agitated with a stirrer. The resulting gel is left in a thermo-hygrostat at 40°C × 60%RH for three hours and then cooled to room temperature. The gel strength of the gel is measured with a curdmeter (Curdmeter-MAX ME-500 manufactured by I. Techno Engineering Co., Ltd).

"Artificial urine" is prepared by mixing urea: 2 wt%, sodium chloride: 0.8 wt%, calcium chloride dihydrate: 0.03 wt%, magnesium sulfate heptahydrate: 0.08 wt%, and ion exchanged water: 97.09 wt%, and the mixture is used at a temperature of 40°C unless otherwise specified.

"Basis weight" is measured as follows. After preliminary drying of a sample or a test piece, the sample or the test piece is left in a test room or a test device under normal conditions (an ambient temperature of 23 ± 1°C and with a relative humidity of 50 ± 2% at the testing site) until the weight of sample or test piece reaches constant mass. Preliminary drying is to achieve the constant mass of the sample or test piece under an environment having a temperature of 100°C. For fibers having a standard moisture regain of 0.0%, preliminary drying may be omitted. The test piece having the constant mass is cut with a cutting template having the size of 100 mm × 100 mm into samples having the size of 100 mm × 100 mm. The weight of the sample is measured. The measured weight is multiplied by 100 to determine the weight per one square meter, which is defined as the basis weight.

"Thickness" is automatically measured with an automatic thickness gauge (KES-G5 handy compression tester) under a load of 0.098 N/cm² and a pressurized area of 2 cm².

"Water absorption capacity" is measured in accordance with JIS K7223-1996 "Testing method for water absorption capacity of super absorbent polymers".

"Water absorption rate" is defined as "time that elapse before the end point" measured with 2g of super absorbent polymers and 50g of normal saline solution in accordance with JIS K7224-1996 "Testing method for water absorption rate of super absorbent polymers".

The tests and measurements are carried out in a laboratory or apparatus under normal conditions (a temperature of 23 ± 1°C and a relative humidity of 50 ± 2% at the testing site), unless the environmental condition for the tests and measurements are otherwise specified.

The dimensions of the components are measured in a spread state, not the natural length state, unless otherwise specified.

### Industrial Applicability

The present invention is applicable to absorbent articles such as disposable diapers and the like.

### Reference Signs List

- 10: top sheet
- 11: back sheet
- 21: absorber
- 60: stretchable sheet
- 61: inner sheet
- 62: outer sheet
- 63: elastically stretchable member (elastic member)
- 65: bonded portion
- 66: non-bonded portion
- 70: lotion portion
- EF: end flap part

## Claims

1. A disposable diaper, comprising:
a liquid pervious top sheet;
a liquid impervious back sheet;
an absorber disposed between the top sheet and the back sheet;
end flap parts formed on both sides of the absorber in a front-back direction; and
a rectangular shaped stretchable sheet stretchable in a width direction provided between the top sheet and the back sheet forming a dorsal side-end flap part,
wherein the stretchable sheet includes an inner sheet opposed to the top sheet, an outer sheet opposed to the back sheet, and elastic members stretching along the width direction and being disposed at predetermined intervals in the front-back direction between the inner sheet and the outer sheet,
the elastic members are fixed via bonded portions formed at predetermined intervals in the width direction on an opposed body side surface of the inner sheet and on a body side surface of the outer sheet,
lotion portions are formed at predetermined intervals in the width direction on a body side surface of the top sheet and extended in the front-back direction from a ventral side-end flap part toward the dorsal side-end flap part, and
in a plan view, that section of the lotion portion which extends in the dorsal side-end flap part is provided such that the section overlaps with one side portion of one bonded portion and with the other side portion of the other bonded portion adjacent to the one bonded portion.

2. The disposable diaper according to claim 1, wherein
a basis weight of lotion applied to the lotion portion is 2 to 20 g/mm².

3. The disposable diaper according to claim 1 or 2, wherein
a basis weight of lotion applied to that section of the lotion portion which extends in the dorsal side-end flap part is larger than a basis weight of lotion applied to that section of the lotion portion which extends on the absorber.

4. The disposable diaper according to any one of claims 1 to 3, wherein
in a plan view, a length in the width direction of that section of the lotion portion which extends in the dorsal side-end flap part is longer than a length in the width direction of that section of the lotion portion which extends on the absorber.

5. The disposable diaper according to any one of claims 1 to 4, wherein
the lotion is formed of water soluble glycerin.
